# EUROPÄISCHE PATENTANMELDUNG

(11) **EP 4 649 858 A2**
(43) Veröffentlichungstag der Anmeldung: **19.11.2025**
(21) Anmeldenummer: 25206455.5
(22) Anmeldetag: 18.06.2021
(51) Int. Cl.: A45D 34/02

(54) **SCHUTZHÜLLE MIT EINER DESINFEKTIONSVORRICHTUNG**

(30) Priorität: 22.06.2020 WO PCT/EP2020/067322
(62) Teilanmeldung aus: 21734118.9
(71) Anmelder: Ammann, Peter, 3653 Oberhofen (CH)
(72) Erfinder: Ammann, Peter, 3653 Oberhofen (CH)
(74) Vertreter: E. Blum & Co. AG

(57) **Zusammenfassung**

Die Erfindung betrifft eine Schutzhülle für eine mobile Kommunikationsvorrichtung, mit einer integrierten Desinfektionsvorrichtung zum Applizieren eines Desinfektionsmittels auf einer Fingerkuppe eines Benutzers. Die Desinfektionsvorrichtung umfasst einen in der Schutzhülle integrierten Behälter für ein Desinfektionsmittel und eine mit dem Behälter fluidverbundene Sprüheinheit. Die Sprüheinheit umfasst einen Sprühkopf und eine mit dem Sprühkopf fluidverbundene Ventilvorrichtung. Die Schutzhülle weist eine durch eine Öffnung von aussen zugängliche Ausnehmung auf, in der der Sprühkopf hineinragt und in der eine Fingerkuppe des Benutzers aufnehmbar ist. Die Ventilvorrichtung ist derart ausgestaltet, dass sie bei Betätigung des Sprühkopfs mittels der Fingerkuppe Desinfektionsmittel aus dem Behälter als Aerosol in die Ausnehmung freigibt, so dass das Aerosol die Fingerkuppe benetzen kann. Der Behälter der Desinfektionsvorrichtung umfasst ein mit dem integrierten Behälter fluidverbundenes Nachfüllventil zum Nachfüllen von Desinfektionsmittel in den Behälter. Das Nachfüllventil ist mit einem flexiblen Schlauch verbunden, der in den integrierten Behälter hineinragt.

## Beschreibung

### Technisches Gebiet

Die vorliegende Erfindung betrifft eine Schutzhülle für eine Kommunikationsvorrichtung mit einer Desinfektionsvorrichtung, gemäss dem unabhängigen Anspruch.

### Hintergrund

Desinfektionsvorrichtungen sind bekannt. Meistens handelt es sich dabei um herkömmliche Sprühdosen die mit einem Desinfektionsmittel gefüllt sind, das beim Drücken des Sprühkopfs freigesetzt wird. Weiter sind auch Spender bekannt, die z.B. stationär in Krankenhäuser, usw. aufgestellt sind und eine relativ grosse Menge Desinfektionsmittel enthalten, damit sie längere Zeit bei der intensiven Verwendung in frequentierten Umgebungen halten. Diese Lösungen haben den Nachteil, dass sie als portable Lösung aufgrund ihrer Grösse nicht geeignet sind. Ausserdem kann die Dosierung schwierig sein und in vielen Fällen wird mit einer Betätigung eine zu grosse Menge des Mittels ausgegeben.

KR 20170116352 offenbart eine Mobiltelefonhülle mit Flüssigkeitsstrahlfunktion, zur Aufnahme verschiedener Flüssigkeiten wie z.B. Handdesinfektionsmittel, wobei die Flüssigkeit gleichzeitig nach aussen gesprüht wird.

### Darstellung der Erfindung

Es stellt sich deshalb die Aufgabe, eine verbesserte Desinfektionsvorrichtung bereitzustellen. Insbesondere stellt sich die Aufgabe, eine Desinfektionsvorrichtung bereitzustellen, die einfach in der Handhabung, effektiv, selektiv einsetzbar, tragbar und wiederverwendbar ist.

Diese Aufgabe wird mit einer Schutzhülle für eine mobile Kommunikationsvorrichtung, insbesondere für ein Mobiltelefon oder einem Tablett, gelöst. Die Schutzhülle umfasst in einem einer Rückseite der Kommunikationsvorrichtung zugewandten Bereich eine integrierte Desinfektionsvorrichtung zum Applizieren eines Desinfektionsmittels auf mindestens einer Fingerkuppe eines Benutzers. Die Desinfektionsvorrichtung umfasst einen in der Schutzhülle integrierten Behälter für ein Desinfektionsmittel, eine mit dem Behälter fluidverbundene Sprüheinheit, wobei die Sprüheinheit einen Sprühkopf und eine mit dem Sprühkopf fluidverbundene Ventilvorrichtung umfasst. Die Schutzhülle weist eine durch eine Öffnung von aussen zugängliche Ausnehmung auf, in der der Sprühkopf hineinragt und in der eine Fingerkuppe des Benutzers aufnehmbar ist. Die Ventilvorrichtung ist derart ausgestaltet, dass sie bei Betätigung des Sprühkopfs mittels der Fingerkuppe Desinfektionsmittel aus dem Behälter als Aerosol in die Ausnehmung freigibt, so dass das Aerosol die Fingerkuppe benetzen kann. Der Behälter der Desinfektionsvorrichtung umfasst ein mit dem integrierten Behälter fluidverbundenes Nachfüllventil zum Nachfüllen von Desinfektionsmittel in den Behälter, wobei das Nachfüllventil mit einem flexiblen Schlauch verbunden ist, der in den integrierten Behälter hineinragt.

Die vorliegende Erfindung hat den Vorteil, dass ein Benutzer die Desinfektionsvorrichtung bei Bedarf in jeder Situation einsetzen kann, bei der im täglichen Leben durch die häufigen unvermeidlichen Berührungen von Gegenständen die Gefahr einer Kontamination und eventuelle Ansteckung mit Erregern besteht, wobei dies schnell und selektiv geschehen kann, indem sie einfach für die Desinfektion eines einzelnen Fingers verwendbar ist und indem eine unerwünschte grossflächige Desinfektion vermieden wird.

Die Erfindung deckt die Desinfektion am Benutzer selbst ab, wobei dieser beispielsweise vor und/oder nach dem Drücken eines Knopfes, z.B. bei Betätigung eines Geldautomats oder von Knöpfen in Aufzügen, wo eine hohe Gefahr der Kontamination mit Erregern besteht, da sie von vielen Personen benutzt werden, den Finger desinfizieren kann, der mit dem Knopf in Kontakt kam. Wird also ein Knopf einer solchen Vorrichtung betätigt, so kann der Benutzer anschliessend die Desinfektionsvorrichtung zum Desinfizieren der Fingerkuppe, die für die Betätigung des Knopfs verwendet wurde, einsetzen. Dafür muss er nur kurz mit der betreffenden Fingerkuppe den Sprühkopf drücken, so dass Desinfektionsmittel die Fingerkuppe benetzen und damit desinfizieren kann. Ausser der offensichtlich einfachen Verwendung hat die Desinfektionsvorrichtung den weiteren wichtigen Vorteil, dass sie selektiv nur für die betroffene Fingerkuppe verwendet werden muss. Wie oben beschrieben geben herkömmliche Vorrichtungen oft zu viel Desinfektionsmittel aus, das in der Hand aufgefangen werden muss, wobei es auch auf den Boden tropfen kann, was eine Verschwendung von Desinfektionsmittel bedeutet. Weiter ist es von Nachteil, dass mit dem flüssigen Mittel in der Hand die ganze Hand bzw. beide Hände durch Einreiben desinfiziert werden muss bzw. müssen. Diese Nachteile vermeidet die vorliegende Desinfektionsvorrichtung, was eine grosse Menge Desinfektionsmittel spart, da gerade für das beschriebene Szenario tatsächlich nur eine kleine Portion einer Hand (die Fingerkuppe) mit dem potentiell kontaminierten Knopf in Berührung kommt und desinfiziert werden muss. Nicht zuletzt ist es aber auch von grossem Vorteil, dass gerade bei häufiger Desinfektion die Austrocknung der Hände, die bekanntlich durch häufiges Desinfizieren der Hände mit typischerweise Alkoholhaltigen Mitteln auftritt, weitgehend vermieden wird.

Die Erfindung hat zusätzlich den Vorteil, dass sie eine tragbare Desinfektionslösung bietet, welche bereits in einem täglich verwendeten Gegenstand integriert ist. Da in der heutigen Zeit ein Mobiltelefon ein ständiger Begleiter ist, führt die Integration der Desinfektionsvorrichtung in der Schutzhülle des Mobiltelefons zu einer effektiven Desinfektionslösung ohne die Notwendigkeit des Mitführens einer zusätzlichen Vorrichtung.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 eine Rückseitenansicht einer erfindungsgemässen Schutzhülle mit einer Desinfektionsvorrichtung, und
Fig. 2 eine Seitenansicht einer erfindungsgemässen Schutzhülle mit einer Desinfektionsvorrichtung.

### Wege zur Ausführung der Erfindung

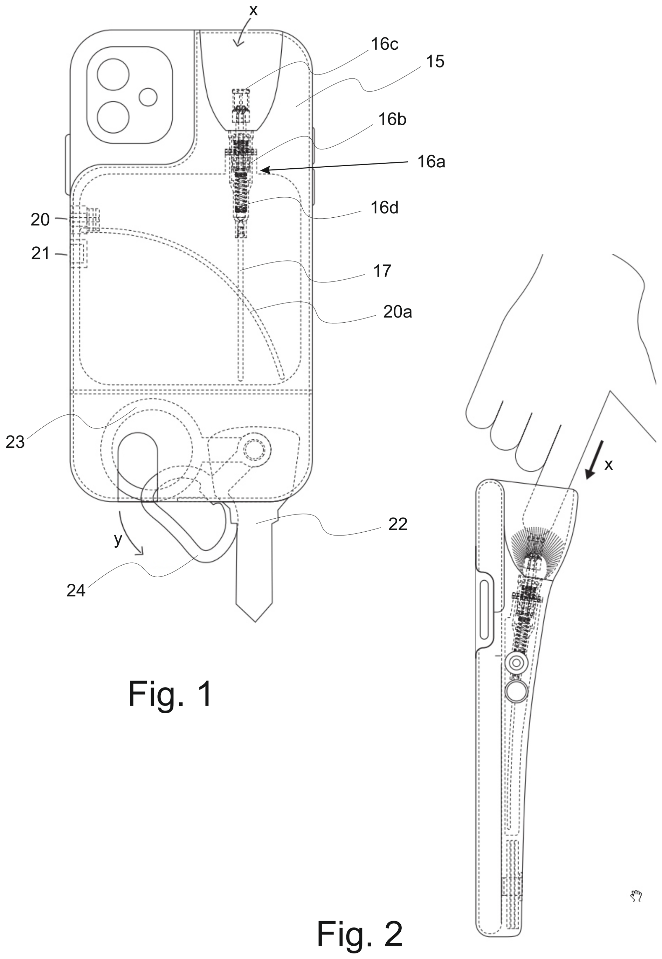

**Fig. 1 und** 2 zeigen eine Rückseitenansicht bzw. eine Seitenansicht einer erfindungsgemässen Schutzhülle 15 mit einer Desinfektionsvorrichtung. Die gestrichelten Linien in den Figuren deuten an, dass sich das betreffende Element im Inneren der Schutzhülle befindet. Wie anfangs erwähnt, ist die Schutzhülle für eine mobile Kommunikationsvorrichtung, insbesondere für ein Mobiltelefon oder einem Tablett, vorgesehen. Sie umfasst in einem einer Rückseite der Kommunikationsvorrichtung zugewandten Bereich eine integrierte Desinfektionsvorrichtung zum Applizieren eines Desinfektionsmittels auf mindestens einer Fingerkuppe eines Benutzers. Die Desinfektionsvorrichtung umfasst einen in der Schutzhülle integrierten Behälter 16a für ein Desinfektionsmittel und eine mit diesem Behälter fluidverbundene Sprüheinheit 16b. Die Sprüheinheit umfasst einen Sprühkopf 16c und eine mit dem Sprühkopf fluidverbundene Ventilvorrichtung 16d. Die Schutzhülle weist eine durch eine Öffnung von aussen zugängliche Ausnehmung auf, in der der Sprühkopf hineinragt und in der eine Fingerkuppe des Benutzers aufnehmbar ist, was mit dem Pfeil x angedeutet ist. Die Ventilvorrichtung ist derart ausgestaltet, dass sie bei Betätigung des Sprühkopfs mittels der Fingerkuppe Desinfektionsmittel aus dem Behälter als Aerosol in die Ausnehmung freigibt, so dass das Aerosol die Fingerkuppe benetzen kann, was in Fig. 2 illustriert ist.

Erfindungsgemäss umfasst der Behälter der Desinfektionsvorrichtung der Schutzhülle weiter ein mit dem Behälter fluidverbundenes Nachfüllventil 20 zum Nachfüllen von Desinfektionsmittel in den integrierten Behälter, wobei das Nachfüllventil mit einem flexiblen Schlauch 20a verbunden ist, der in den integrierten Behälter hineinragt. Das Nachfüllventil ist derart ausgestaltet, dass es von einem geeigneten Aufsatz eines Nachfüllbehälters zum Befüllen des integrierten Behälters geöffnet werden kann und sich selbsttätig wieder schliesst, sobald der Nachfüllbehälter entfernt wird. Mit Vorteil kann auf diese Weise die Desinfektionsvorrichtung der Schutzhülle wieder befüllt werden.

Alternativ oder zusätzlich umfasst der Behälter der Desinfektionsvorrichtung der Schutzhülle einen Nachfüllverschluss 21, welcher zum Nachfüllen von Desinfektionsmittel in den Behälter eine Einfüllöffnung des Behälters ventilfrei freigibt. Mit Vorteil ermöglicht diese Variante das Nachfüllen des Behälters aus jedem beliebigen Nachfüllbehälter, ohne einen speziellen ventilöffnenden Aufsatz.

Der Behälter der Desinfektionsvorrichtung der Schutzhülle umfasst vorzugsweise weiter ein Entlüftungsventil das den integrierten Behälter zur Entlüftung mit der Umgebung verbindet (nicht gezeigt).

Die Ausnehmung ist vorzugsweise mit einem Schwammelement gefüllt (nicht gezeigt), in welchem der Sprühkopf komplett eingebettet ist. Das Schwammelement ist auch vorzugzugsweise austauschbar.

Die Schutzhülle umfasst weiter vorzugsweise eine Abdeckkappe (nicht gezeigt) für die Ausnehmung, so dass der Sprühkopf geschützt ist und nicht aus Versehen betätigt wird.

Die Schutzhülle ist in Ausführungsformen mindestens im Bereich des integrierten Behälters zur Aufnahme des integrierten Behälters doppelwandig. Dies ist vorteilhaft als zusätzliche Sicherheit gegen Auslaufen des Desinfektionsmittels. Der integrierte Behälter kann in dieser Ausführungsform vorzugsweise austauschbar sein. Alternativ ist die Schutzhülle mindestens bereichsweise doppelwandig und ein entsprechender Zwischenraum bildet selbst den integrierten Behälter. Mit Vorteil ist bei dieser Alternative kein zusätzlicher Behälter nötig, wobei die Schutzhülle in diesem Fall selbstverständlich aus einem Material gefertigt ist, das für das Desinfektionsmittel undurchlässig ist.

Weiter umfasst die Schutzhülle vorzugsweise eine Kammer für die Aufbewahrung von Schlüssel 22 oder Getränkeöffner 24 oder ähnliches, welche zur Verwendung herausgeklappt werden können (mit dem Pfeil y angedeutet).

Weiter kann die Schutzhülle einen Ring 23 umfassen, wobei der Ring einen derartigen Durchmesser hat, dass er über einen Türgriff gezogen werden kann, um den Griff ohne direkten Kontakt durch den Benutzer zu betätigen. Weiter ist der Ring so ausgestaltet, dass er keine Kratzer am betätigten Griff hinterlässt. Dazu kann er beispielsweise mit einer Gummischicht beschichtet sein. Die Verwendung eines solchen Rings ist vorteilhaft für das Szenario der Desinfektion von Türgriffen, da diese sehr unterschiedliche Formen haben können und z.B. vergleichsweise stark gebogen sein und stark variierende Durchmesser haben können. Daher ist der Durchmesser des Rings sehr grosszügig gewählt, damit möglichst jede Art von Griff vom Ring erfasst werden kann. Ausserdem ist es auf diese Weise sehr leicht den Ring über den Griff zu ziehen.

Die vorliegende Erfindung hat eine Vielzahl von Vorteilen gegenüber bereits bekannten Lösungen. Sie bietet eine grosse Flexibilität der Anwendung bei unterschiedlichsten Desinfektionsszenarien, da die Desinfektionsvorrichtung in einer Schutzhülle für ein mobiles Kommunikationsgerät eingebettet und somit tragbar ist. Mit der Erfindung kann Desinfektionsmittel gespart werden und die Desinfektion am Benutzer selbst kann schonender für die Hände durchgeführt werden, indem lediglich die betreffenden Fingerkuppen, die tatsächlich Kontakt mit einem fremden Gegenstand hatten, punktuell desinfiziert werden, so dass die Haut der restlichen Hand nicht angegriffen wird.

Während in der vorliegenden Anmeldung bevorzugte Ausführungen der Erfindung beschrieben sind, ist klar darauf hinzuweisen, dass die Erfindung nicht auf diese Beschränkt ist und in auch anderer Weise innerhalb des Umfangs der folgenden Ansprüche ausgeführt werden kann.

## Patentansprüche

1. Schutzhülle für eine mobile Kommunikationsvorrichtung, insbesondere für ein Mobiltelefon oder einem Tablett, wobei die Schutzhülle in einem einer Rückseite der Kommunikationsvorrichtung zugewandten Bereich eine integrierte Desinfektionsvorrichtung zum Applizieren eines Desinfektionsmittels auf mindestens einer Fingerkuppe eines Benutzers umfasst, wobei die Desinfektionsvorrichtung folgendes umfasst:
- einen in der Schutzhülle integrierten Behälter für ein Desinfektionsmittel,
- eine mit dem Behälter fluidverbundene Sprüheinheit, wobei die Sprüheinheit einen Sprühkopf und eine mit dem Sprühkopf fluidverbundene Ventilvorrichtung umfasst,
wobei die Schutzhülle eine durch eine Öffnung von aussen zugängliche Ausnehmung aufweist, in der der Sprühkopf hineinragt und in der eine Fingerkuppe des Benutzers aufnehmbar ist,
wobei die Ventilvorrichtung derart ausgestaltet ist, dass sie bei Betätigung des Sprühkopfs mittels der Fingerkuppe Desinfektionsmittel aus dem Behälter als Aerosol in die Ausnehmung freigibt, so dass das Aerosol die Fingerkuppe benetzen kann, **dadurch gekennzeichnet, dass** der Behälter der Desinfektionsvorrichtung ein mit dem integrierten Behälter fluidverbundenes Nachfüllventil zum Nachfüllen von Desinfektionsmittel in den Behälter umfasst, wobei das Nachfüllventil mit einem flexiblen Schlauch verbunden ist, der in den integrierten Behälter hineinragt.

2. Schutzhülle nach Anspruch 1, wobei der Behälter der Desinfektionsvorrichtung einen Nachfüllverschluss umfasst, welcher zum Nachfüllen von Desinfektionsmittel in den Behälter eine Einfüllöffnung des Behälters ventilfrei freigibt.

3. Schutzhülle nach einem der Ansprüche 1 bis 2, wobei der Behälter der Desinfektionsvorrichtung ein Entlüftungsventil umfasst, das den integrierten Behälter zur Entlüftung mit der Umgebung verbindet.

4. Schutzhülle nach einem der Ansprüche 1 bis 3, wobei die Ausnehmung mit einem Schwammelement gefüllt ist, in welchem der Sprühkopf komplett eingebettet ist, insbesondere wobei das Schwammelement austauschbar ist.

5. Schutzhülle nach einem der Ansprüche 1 bis 4, weiter umfassend eine Abdeckkappe für die Ausnehmung.

6. Schutzhülle nach einem der Ansprüche 1 bis 5, wobei die Schutzhülle mindestens im Bereich des integrierten Behälters zur Aufnahme des integrierten Behälters doppelwandig ist oder wobei die Schutzhülle mindestens bereichsweise doppelwandig ist und ein entsprechender Zwischenraum selbst den integrierten Behälter bildet.
